Europäisches Patentamt

European Patent Office

Office européen des brevets

(11) Publication number: **0 421 581 A1**

# EUROPEAN PATENT APPLICATION

(21) Application number: **90308398.8**

(22) Date of filing: **31.07.90**

(51) Int. Cl.⁵: **A61K 9/50, A61K 9/52**

(30) Priority: **03.10.89 US 416577**

(43) Date of publication of application:
**10.04.91 Bulletin 91/15**

(84) Designated Contracting States:
**BE DE DK ES FR GB GR IT**

(71) Applicant: **WARNER-LAMBERT COMPANY**
**201 Tabor Road**
**Morris Plains New Jersey 07950(US)**

(72) Inventor: **Tai, Anna W.**
**225 Longview Road**
**Bridgewater, New Jersey 08807(US)**

(74) Representative: **Bond, Bentley George et al**
**HASELTINE LAKE & CO. Hazlitt House 28**
**Southampton Buildings Chancery Lane**
**London, WC2A 1AT(GB)**

(54) Chewable spray dried spheroidal microcapsules and wax coated microcapsules and methods for preparing same.

(57) The present invention pertains to a spray dried spheroidal microcapsule under about 150 microns in diameter which comprises (a) a medicament present in an amount from about 1% to about 90%, by weight of the microcapsule composition, (b) a film forming polymer present in an amount from about 8% to about 90%, by weight of the microcapsule composition, (c) a plasticizing agent in an amount from about 5% to about 30%, by weight of the film forming polymer. In another embodiment, the invention is directed at a spheroidal coated microcapsule under about 850 microns in diameter which comprises at least one spray dried spheroidal microcapsule core under about 150 microns in diameter and a coating layer over the core, wherein the coating layer comprises in percentages by weight of the coating layer composition (a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%, and (b) a surface active agent present in an amount from about 0.5% to about 20%.

EP 0 421 581 A1

## CHEWABLE SPRAY DRIED SPHEROIDAL MICROCAPSULES AND WAX COATED MICROCAPSULES AND METHODS FOR PREPARING SAME

This invention pertains to novel chewable spray dried spheroidal microcapsule and coated microcapsule compositions. The novel microcapsule compositions comprise a medicament, a film forming polymer, and a plasticizing agent. The microcapsules may be coated with an edible material selected from the group consisting of (i) fatty acids, (ii) natural waxes, (iii) synthetic waxes, and (iv) mixtures thereof. Therapeutically effective amounts of the spray dried spheroidal microcapsules and spheroidal coated microcapsules may be utilized in a wide variety of pharmaceutically acceptable carriers and confectionery bulking agents to prepare medicated products and sustained release medicated compositions. This invention also relates to methods for preparing these chewable spray dried spheroidal microcapsules and spheroidal coated microcapsules and the medicated products and the sustained release medicated compositions in which they may be used.

Sustained release compositions for the sequential or timed release of medicaments are well known in the art. Generally such compositions contain medicament particles, normally administered in divided doses two or more times daily, mixed with or covered by a coating material which is resistant to degradation or disintegration in the stomach and/or in the intestine for a selected period of time. Release of the medicament may occur by leaching, erosion, rupture, diffusion or similar actions, depending upon such factors as the nature and thickness of the coating material.

Sustained release formulations initially utilized solvent-based film coatings. Because of cost and environmental and safety (toxicity) concerns with the solvents, colloidal (latex) and near colloidal (pseudolatex) aqueous dispersion coatings were developed to replace solvent-based film coatings.

Latex dispersions, such as those disclosed in United States patent no. 3,608,063, issued to Banker, and United States patent no. Re. 28,316, also issued to Banker et al. and assigned to Purdue Research Foundation, are made from a synthetic polymer with a liquid monomer and are prepared by emulsion polymerization. Pseudolatex dispersions can be prepared from any thermoplastic, water-insoluble polymer. Preferred pseudolatex polymers for use in many pharmaceutical applications are the derivatives of cellulose.

A frequently encountered problem in the field of chewable sustained release compositions is unsuitable particle size and shape. Particle sizes larger than about 850 microns are usually considered unsatisfactory for chewing because these particles are gritty and are easily broken during chewing thereby causing an off-taste and premature release of the medicament in the mouth. Spheroidal sustained release particles are generally preferred over nonspheroidal particles because these uniformly coated materials protect the medicament from premature release and release the medicament more uniformly.

Small sustained release particles, or microcapsules, suitable for use in chewing compositions are generally easier to prepare with a solvent-based film coating because of the high volatility and low surface tension of the solvent. For the reasons set out above, sustained release microcapsules prepared from aqueous-based coating materials are usually preferred, however, these microcapsules are generally larger in size than those obtained from solvent-based coating materials and must be ground to obtain smaller chewable microcapsules. These ground smaller particles are usually not satisfactory for use in sustained release compositions because the particles are irregular, are not spheroidal and do not release the medicament uniformly.

United States patent no. 4,749,575, issued to Rotman and assigned to Bio-Dar Ltd., discloses the preparation of chewable microcapsules of less than 300 microns diameter formed by dissolving a polymer such as hydroxypropyl methylcellulose phthalate, hydroxyphenyl methylcellulose or various acrylic resins in an organic solvent and coating a medicament with the polymer solution in a fluidized bed.

European patent application no. 266,113 discloses a method for preparing a taste masked therapeutic composition which comprises spray drying a suspension of acetoaminophen in a solution of an acrylic polymer in an organic solvent.

European patent application no. 250,648 and Goodman et al., Journal of Pharmaceutical Sciences , 59 , 1131-1137 (1970), discloses methods for preparing microspheres suitable for formulating into a tablet which comprise slurrying an aqueous mixture of a drug and an acrylic polymer, vacuum drying the mixture, then grinding the so-formed particles, and compressing the particles into a tablet.

PCT application no. PCT/U.S.87/03068 discloses a method for preparing a taste-masked pharmaceutical composition which comprises spraying in a fluidized bed a suspension comprised of a mixture of an aqueous based solution of a high temperature film forming polymer and a low temperature film forming polymer onto particles of a pharmaceutical core material. The high temperature film forming polymer can be ethyl cellulose or an acrylic polymer and the low temperature film forming polymer can be an acrylic polymer.

European patent application no. 265,226 discloses a method for preparing a taste masked therapeutic composition which comprises spray drying a suspension of colloidal silica in an alcoholic solution of acetoaminophen and ethyl cellulose.

United States patent no. 4,764,380, issued to Urquhart et al. and assigned to Alza Corporation, discloses a drug delivery system for microcapsules which can be prepared from an aqueous mixture of the drug and ethyl cellulose. The mixture is blended and kneaded, then extruded and passed through a 20 mesh screen. The microcapsules may be coated with ethyl cellulose by air suspension.

United States patent no. 4,597,970, issued to Sharma et al. and assigned to Warner-Lambert Company, discloses a chewing gum composition comprising an agglomerated sweetener delivery system comprising a sweetener core material and a hydrophobic matrix consisting essentially of (a) lecithin, (b) an edible material selected from the group consisting of (i) fatty acids, (ii) natural waxes, (iii) synthetic waxes, and (iv) mixtures thereof, and (c) at least one glyceride.

While the above sustained release compositions provide some degree of improved chewable sustained release activity, none of the above compositions are entirely satisfactory. Chewable sustained release compositions prepared from organic solvent based polymer solutions are expensive and pose environmental and toxicity problems. Chewable sustained release compositions prepared from aqueous dispersions of polymers generally provide large microcapsules which must be ground to smaller particles which are usually not uniform in size, shape and composition. Thus it would be advantageous to prepare a sustained release composition from an aqueous dispersion of polymer whereby the microcapsules formed are spheroidal and are of sufficiently small size such that they are not gritty and can be chewed without being broken. The present invention provides such improved chewable spheroidal microcapsules and spheroidal coated microcapsules without the disadvantages characteristic of previously known products. The present invention also provides methods for preparing these improved spheroidal microcapsules and coated microcapsules and the medicated products and sustained release compositions in which they may be employed.

The present invention pertains to spray dried spheroidal microcapsules under about 150 microns in diameter which comprise (a) a medicament present in an amount from about 1% to about 90%, by weight of the microcapsule composition, (b) a film forming polymer present in an amount from about 8% to about 90%, by weight of the microcapsule composition, and (c) a plasticizing agent in an amount from about 5% to about 30%, by weight of the film forming polymer. In another embodiment, the invention is directed at spheroidal coated microcapsules under about 850 microns in diameter which comprise at least one spray dried spheroidal microcapsule core under about 150 microns in diameter and a coating layer over the core, wherein the coating layer comprises in percentages by weight of the coating layer composition (a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%, and (b) a surface active agent present in an amount from about 0.5% to about 20%. The microcapsule and coated microcapsule compositions may be utilized in a wide variety of pharmaceutically acceptable carriers and confectionery bulking agents to prepare medicated products and sustained release compositions. This invention also relates to methods for preparing these microcapsules and the medicated products and sustained release compositions in which they may be employed.

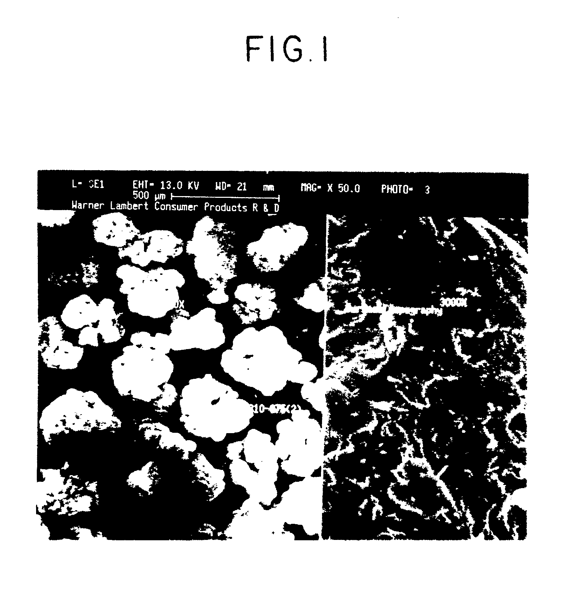

FIGURE 1 is a photomicrograph of spray dried spheroidal microcapsules of pseudoephedrine sulfate and ethyl cellulose film coated with wax by air suspension (left half, magnification 50X, right half, magnification 3000X). The spheroidal appearance is clearly seen.

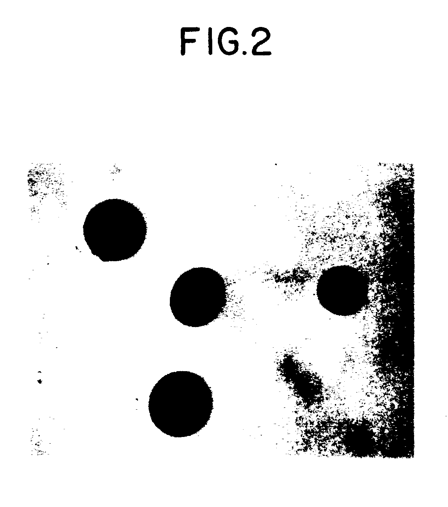

FIGURE 2 is a photomicrograph depicting spray dried spheroidal microcapsules of pseudoephedrine sulfate and ethyl cellulose film coated with wax by spray congealing (left half, magnification 50X, right half, magnification 100X). The spheroidal appearance is clearly seen.

FIGURE 3 depicts in graphic format the sustained release properties versus time of wax coated microcapsules containing pseudoephedrine sulfate and ethyl cellulose.

The present invention pertains to improved chewable medicated products and sustained release compositions for administering a medicament, normally administered in divided doses two or more times daily. More specifically, the present invention pertains to spray dried spheroidal microcapsules under about 150 microns in diameter which comprise (a) a medicament present in an amount from about 1% to about 90%, by weight of the microcapsule composition, (b) a film forming polymer present in an amount from about 8% to about 90%, by weight of the microcapsule composition, and (c) a plasticizing agent in an amount from about 5% to about 30%, by weight of the film forming polymer.

In another embodiment, the invention is directed at spheroidal coated microcapsules under about 850 microns in diameter which comprise at least one spray dried spheroidal microcapsule core under about 150 microns in diameter and a coating layer over the core, wherein the coated microcapsules comprise (A) a microcapsule core comprising (a) a medicament present in an amount from about 1% to about 90%, by weight of the core composition, (b) a film forming polymer present in an amount from about 8% to about 90%, by weight of the core composition, and (c) a plasticizing agent in an amount from about 5% to about 30%, and (B) a coating layer over the core comprising in percentages by weight of the coating layer composition (a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%, and (b) a surface active agent present in an amount from about 0.5% to about 20%.

The microcapsule and coated microcapsule compositions may be utilized in a wide variety of pharmaceutically acceptable carriers and confectionery bulking agents to prepare medicated products and sustained release compositions. This invention also relates to methods for preparing these microcapsules and coated microcapsules and the medicated products and sustained release compositions in which they may be employed.

While the invention is not to be limited to theoretical considerations, applicant believes that by carefully controlling the concentration of a medicament and a film forming polymer in an aqueous suspension, and carefully controlling the conditions of spray drying the suspension, spheroidal microcapsules of sufficiently small particle size suitable for use in chewable medicated compositions can be prepared. By forming the microcapsules by spray drying, applicant's microcapsules are spheroidal and release the medicament more uniformly than prior art compositions. Moreover because applicant's microcapsule compositions are prepared from aqueous slurries, the resulting compositions are more economical to prepare and do not have the potential toxicity and environmental problems which can accompany the preparation of such compositions from organic solutions. Furthermore by carefully controlling the conditions for coating the spheroidal microcapsules with an edible material, spheroidal coated microcapsules or spheroidal coated agglomerates of microcapsules, which contain a plurality of microcapsules in a single coating, can also be prepared suitable for use in chewable sustained release medicated compositions.

In a preferred embodiment, the spray dried spheroidal microcapsules comprise (a) a medicament present in an amount from about 1% to about 90%, by weight of the microcapsule composition, (b) a film forming polymer present in an amount from about 8% to about 90%, by weight of the microcapsule composition, and (c) a plasticizing agent in an amount from about 5% to about 30%, by weight of the film forming polymer. In a more preferred embodiment, the spray dried spheroidal microcapsules comprise in percentages by weight of the microcapsule composition (a) a medicament present in an amount from about 1% to about 70%, by weight of the microcapsule composition, (b) a film forming polymer present in an amount from about 20% to about 90%, by weight of the microcapsule composition, and (c) a plasticizing agent in an amount from about 5% to about 24%, by weight of the film forming polymer. In a most preferred embodiment, the spray dried spheroidal microcapsules comprise in percentages by weight of the microcapsule composition (a) a medicament present in an amount from about 1% to about 50%, by weight of the microcapsule composition, (b) a film forming polymer present in an amount from about 30% to about 90%, by weight of the microcapsule composition, and (c) a plasticizing agent in an amount from about 5% to about 20%, by weight of the film forming polymer.

In an alternative embodiment, the spray dried spheroidal microcapsules may further comprise a dispersing agent present in an amount up to about 12%, preferably in an amount up to about 10%, and more preferably in an amount up to about 7%, by weight of the microcapsule composition.

The medicaments (drugs, pharmaceuticals) of the present invention may be selected from a wide variety of water-soluble and water-insoluble drugs and their acid addition salts. Both organic and inorganic salts may be used provided the drug maintains its medicament value. Exemplary acid salts include hydrochloride, hydrobromide, orthophosphate, benzoate, maleate, tartrate, succinate, citrate, salicylate, sulfate and acetate.

The medicament may be selected from a wide range of therapeutic agents and mixtures of therapeutic agents which may be administered in sustained release or prolonged action form. Nonlimiting illustrative categories and specific of such medicaments include:

(a) Analgesics, such as acetylsalicylic acid, acetaminophen, ibuprofen, phenacetin, phenylbutazone, salicylamide, sodium salicylate, and meclofenamic acid;

(b) Anthelmintics, such as dithiazanine iodide and gardona;

(c) Antiasmatics, such as aminophylline, metaproterenol, epinephrine and theophylline;

(d) Anticholesterolemic and antilipid agents, such as gemfibrozil;

(c) Antiemetics, such as prochloroperazine dimaleate;

(f) Antihistamines, such as chlorpheniramine maleate, brompheniramine maleate, phenindamine tartrate, pyrilamine maleate, methapyrilene fumarate, doxylamine succinate, phenyltoloxamine citrate, diphenylhydramine hydrochloride, promethazine, terfenedine and triprolidine;

(g) Anti-inflammatory agents, such as isoxicam, meclophenamic acid and naproxen;

(h) Antinauseants, such as dimenhydrinate and meclizine;

(i) Antipyretics, such as N-acetyl-p -aminophenol;

(j) Antitussives, such as dextromethorphan, dextromethorphan hydrobromide, noscapine, carbetapentane citrate, chlophedianol hydrochloride, codeine and diphenhydramine hydrochloride;

(k) Appetite suppressants, such as phenylpropanolamine hydrochloride and caffeine;

(l) Cathartics, such as castor oil;

(m) Central nervous system stimulants, such as nicotine and caffeine;

(n) Decongestants, such as phenylephrine hydrochloride, phenylpropanolamine hydrochloride, pseudoephedrine hydrochloride, pseudoephedrine hydrobromide, pseudoephedrine sulfate and ephedrine;

(o) Expectorants, such as guaifenesin and glycerol guaiacolate;

(p) Laxatives, such as phenolphthalein, danthron, pamabrom and bisocadyl;

(q) Nutritional supplements, including vitamins and minerals, such as ascorbic acid, niacin, pantothenic acid, vitamin B6, thiamine hydrochloride, riboflavin, potassium iodide, potassium chloride, cupric sulfate and ferrous sulfate; and

(r) Various alkaloids, such as codeine phosphate, codeine sulfate and morphine.

In a preferred embodiment, the medicament is a water-soluble medicament selected from the group consisting of dextromethorphan, dextromethorphan hydrobromide, pseudoephedrine, pseudoephedrine sulfate, pseudoephedrine hydrochloride, pseudoephedrine hydrobromide, chlorpheniramine maleate, guaifenesin, diphenhydramine hydrochloride, and mixtures thereof. In a more preferred embodiment the medicament is a water-soluble medicament selected from the group consisting of pseudoephedrine sulfate, diphenhydramine hydrochloride, chlorpheniramine maleate, and mixtures thereof.

The medicament of the present invention may be used in many distinct physical forms well known in the pharmaceutical art to provide an initial dosage of the medicament and/or a further time-release form of the medicament. Without being limited thereto, such physical forms include free forms and encapsulated forms, and mixtures thereof.

The amount of medicament drug or its acid addition salt used in the present invention may vary depending upon the therapeutic dosage recommended or permitted for the particular medicament. In general, the amount of medicament present is the ordinary dosage required to obtain the desired result. Such dosages are known to the skilled practitioner in the medical arts and are not a part of the present invention.

The film forming polymers useful in the present invention are aqueous colloidal dispersions containing spherical, solid or semisolid particles less than about one (1) micron in diameter, and typically less than about 0.1 micron in diameter. Aqueous colloidal mixtures are generally very fluid at concentrations from about 20% to about 40%. Suitable film forming polymers in the present invention include cellulose derivatives such as ethylcellulose, hydroxypropylcellulose, hydroxypropylmethylcellulose, hydroxypropylmethylcellulose phthalate, methylcellulose, sodium carboxymethylcellulose, and the like, and mixtures thereof. Other suitable film forming polymers in the present invention include aqueous acrylic resin dispersions such as polyacrylamide, polyacryldextran, polyalkyl cyanoacrylate, polymethyl methacrylate, and the like, and mixtures thereof. In a preferred embodiment, the film forming polymer is a cellulose derivative such as ethylcellulose. In a more preferred embodiment, the film forming polymer is the commercially available aqueous polymeric dispersion manufactured under the tradename AQUACOAT (24.5%-30% ethyl cellulose), by FMC Corporation, Princeton, New Jersey. In another more preferred embodiment, the film forming polymer is the commercially available aqueous polymeric dispersion manufactured under the tradename SURELEASE (containing 24.5%-29.5% ethyl cellulose, dibutyl sebacate and oleic acid as plasticizing agents, and fumed silica as an anti-adherent), by Colorcon, Inc., West Point, Pennsylvania.

Plasticizing agents (plasticizers) are organic molecules added to a polymer to facilitate processing and to increase the flexibility and toughness of the final product by internally modifying (solvating) the polymer molecule. Plasticizers should be soluble in the polymer they are designed to plasticize, should not be highly water-soluble, and should be safe for the intended use. Suitable plasticizing agents in the present invention are nonvolatile organic liquids and low melting solids, such as esters of phthalic acid, adipic acid and sebacic acid, and polyols such as ethylene glycol and its derivatives, tricresyl phosphate, castor oil, and the like, and mixtures thereof. Other suitable partly water-soluble to water-insoluble plasticizing agents that may be incorporated in cellulosic dispersion products include dibutyl sebacate triethyl citrate, tributyl citrate, triacetin, and acetylated mono-, di- and triglycerides, and the like, and mixtures thereof. Other suitable plasticizing agents include acetyltriethylcitrate, triethyl-citrate, acetyltributylcitrate and tributylcitrate, and the like, and mixtures thereof. In a preferred embodiment, the plasticizing agent is acetyltributylcitrate, dibutyl sebacate, and mixtures thereof. In a more preferred embodiment, the plasticizing agent is dibutyl sebacate.

The amount of plasticizing agent present in the microcapsule composition will depend upon the amount of film forming polymer present in the microcapsule composition. Hence the amount of plasticizing agent present is set out above as "by weight of the film forming polymer." The amount of plasticizing agent present by weight of the microcapsule composition may be determined by multiplying the percentage of plasticizing agent present, by weight of the film forming polymer, by the percentage of film forming polymer present, by weight of the microcapsule composition. For example, when the amount of plasticizing agent present in the microcapsule composition is 5%, by weight of the film forming polymer, and the amount of film forming polymer present in the microcapsule composition is 8%, by weight of the microcapsule composition, the amount of plasticizing agent present by weight of the microcapsule composition will be 0.4% (5% of 8%).

Dispersing agents are surface-active agents added to a suspending medium to promote uniform separation of extremely fine (colloidal) solid particles. Suitable dispersing agents, when present, include polymeric electrolytes, condensed silicates, polyphosphates, lignin derivatives including aluminum stearate, aluminum laurate, magnesium stearate, calcium stearate, zinc stearate, talc, kaolin, fumed silica, and the like, and mixtures thereof. In a preferred embodiment, the dispersing agent is selected from the group consisting of aluminum stearate, kaolin, fumed silica, and mixtures thereof. In a more preferred embodiment, the dispersing agent is aluminum stearate.

The microcapsules of the present invention have a diameter under about 150 microns, preferably under about 100 microns, and most preferably under about 50 microns. The spray dried microcapsules formed in the present invention are spheroidal, that is, the microcapsules formed resemble spheres.

In another embodiment, the invention is directed at spheroidal coated microcapsules under about 850 microns in diameter which comprise at least one spray dried spheroidal microcapsule core under about 150 microns in diameter and a coating layer over the core, wherein the coated microcapsules comprise:

(A) a microcapsule core comprising:

(a) a medicament present in an amount from about 1% to about 90%, by weight of the core composition;

(b) a film forming polymer present in an amount from about 8% to about 90%, by weight of the core composition; and

(c) a plasticizing agent in an amount from about 5% to about 30%, by weight of the film forming agent; and

(B) a coating layer over the core comprising in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%.

The fatty acid or wax edible material in the coating layer is preferably present in the range from about 80% to abut 99.5%, more preferably from about 85% to about 99.5%, and most preferably from about 90% to about 99.5%, by weight of the coating layer composition. These amounts are necessary to adequately coat the microcapsule and provide sustained release properties. The surface active agent in the coating layer is preferably present in the range from about 0.5% to about 20%, more preferably from about 0.5% to about 15%, and most preferably from about 0.5% to about 10%, by weight of the coating layer composition.

The edible material in the coating layer is a material which has a melting point in the range from about 25° C. to about 100° C., preferably from about 35° C. to abut 100° C., and more preferably from about 45° C. to about 100° C. The melting point of the edible material should be within the recited range because the melting point of the final coated microcapsule product will be greatly affected by the fat or wax constituent.

The materials useful in the coating layer are selected from the group consisting of fatty acids, natural waxes, synthetic waxes, and the like, and mixtures thereof. Fatty acids are carboxylic acids derived from or contained in an animal or vegetable fat or oil. Fatty acids are composed of a chain of alkyl groups containing from 4 to 22 carbon atoms and are characterized by a terminal carboxyl group. Waxes are low-melting organic mixtures or compounds having a high molecular weight, are solid at room temperature and generally are similar in composition to fats and oils except that waxes contain no glycerides. Waxes may be hydrocarbons or esters of fatty acids and alcohols. Fatty acids and waxes are both classified as lipids.

The fatty acids useful in the present invention are acids which have an iodine value from about 1 to about 10. The iodine value is a means of determining the degree of unsaturation in a fat or oil. The measurement of iodine values is determined by known titrating methods and is reported in terms of centigrams of iodine absorbed per gram of fat or oil sample titrated. (See "Bailey's Industrial Oil and Fat Products," Vol. 2, 4th Ed., Swern, Daniel ed., pp. 436-438 (1982)). Hence the fatty acids useful in the present invention have an iodine value from abut 1 centigram to about 10 centigrams.

Fatty acids useful in the present invention are selected from the group consisting of hydrogenated palm oil, hydrogenated palm kernel oil, hydrogenated peanut oil, hydrogenated rapeseed oil, hydrogenated rice bran oil, hydrogenated soybean oil, hydrogenated cottonseed oil, hydrogenated sunflower oil, hydrogenated castor oil, and the like, and mixtures thereof. Other fatty acids include, for example, decenoic acid, docosanoic acid, stearic acid, palmitic acid, lauric acid, myristic acid, and the like, and mixtures thereof. The preferred fatty acids are selected from the group consisting of hydrogenated palm oil, hydrogenated castor oil, hydrogenated cottonseed oil, stearic acid, palmitic acid, and mixtures thereof. The most preferred fatty acid is stearic acid.

Waxes useful in the present invention include natural waxes, such as animal waxes, vegetable waxes, and petroleum waxes (i.e., paraffin waxes, microcrystalline waxes, petrolatum waxes, mineral waxes), and synthetic waxes which are edible and have a melting point within the range from about 25° C. to about 100° C. Specific examples of useful waxes are spermaceti wax, carnauba was, Japan wax, bayberry wax, flax wax, beeswax, Chinese wax, shellac wax, lanolin wax, sugarcane wax, candelilla wax, paraffin wax, microcrystalline wax, petrolatum wax, carbowax, and the like, and mixtures thereof. Mixtures of these waxes with the fatty acids set out above may also be used. The preferred waxes are selected from the group consisting of carnauba wax, bees wax, glyceryl tristearate, glyceryl monostearate, paraffin wax, microcrystalline wax, glyceryl distearate, and mixtures thereof. The most preferred waxes are carnauba wax, bees wax, glyceryl tristearate, glyceryl monostearate, and paraffin wax.

The wax may also be an ester of a fatty acid having from about 12 to about 31 carbon atoms and a fatty alcohol having from about 12 to about 31 carbon atoms, the ester having a carbon atom content from about 24 to about 62 carbon atoms. Examples of such fatty acid esters are myricyl palmitate, ceryl palmitate, ceryl cerotate, myricyl melissate, stearyl palmitate, stearyl myristate, lauryl laurate, and the like, and mixtures thereof. The preferred fatty acid esters are selected from the group consisting of stearyl palmitate, stearyl myristate, and mixtures thereof.

The wax may also be a monoglyceryl ester, diglyceryl ester, or triglyceryl ester (glycerides) which is an ester formed from a fatty acid having from about 10 to about 22 carbon atoms and glycerol, wherein one or more of the hydroxyl groups of glycerol is substituted by a fatty acid. Examples of useful glycerides include glyceryl monostearate, glyceryl distearate, glyceryl tristearate, glyceryl dipalmitate, glyceryl tripalmitate, glyceryl monopalmitate, glyceryl dilaurate, glyceryl trilaurate, glyceryl monolaurate, glyceryl didocosanoate, glyceryl tridocosanoate, glyceryl monodocosanoate, glyceryl monocaproate, glyceryl dicaproate, glyceryl tricaproate, glyceryl monomyristate, glyceryl dimyristate, glyceryl trimyristate, glyceryl monodecenoate, glyceryl didecenoate, glyceryl tridecenoate, and the like, and mixtures thereof. The preferred glycerides are selected from the group consisting of glyceryl monostearate, glyceryl distearate, glyceryl tristearate, and mixtures thereof.

The surface active agent in the coating layer in this invention aids in dispersing immiscible components into a single stable system. Surface active agents (surfactants, wetting agents, emulsifiers) are compounds which, inter alia, reduce interfacial tension between a liquid and a solid. Surface active agents useful in this invention include acetylated monoglycerides (cetodan), stearic acid, oleic acid, polyethylene glycol, glyceryl monostearate, lecithin, fatty acid monoglycerides (dimodan), diglycerides, propylene glycol monostearate, lecithin, and the like, and mixtures thereof. The preferred surface active agents are selected from the groups consisting of glyceryl monostearate, acetylated monoglycerides, stearic acid, and the like, and mixtures thereof. The more preferred surface active agent is glyceryl monostearate.

The weight ratio of microcapsule core composition to coating layer composition is the ratio containing sufficient coating layer to prevent potential premature release of the medicament from the microcapsule core without forming a composition so large so as to be therapeutically unsuitable for use in chewable compositions. In general, the weight ratio of microcapsule core composition to coating layer composition is from about 1:9 to about 9:1, preferably from about 1:4 to about 1:1, and more preferably from about 1:4 to about 1:2, respectively.

The spheroidal coated microcapsules of the present invention have a diameter under about 850 microns, preferably under about 750 microns, and most preferably under about 650 microns. The spheroidal coated microcapsules may also be agglomerates of coated microcapsules which may contain one or more core microcapsule gathered into a cluster under a single coating layer. The terms "coated microcapsules" and "agglomerates of coated microcapsules" are used interchangeably herein.

The present invention is also directed at methods for preparing the spray dried spheroidal microcapsules and coated microcapsules. More particularly, the present invention is directed at a method for preparing spray dried spheroidal microcapsules under about 150 microns in diameter, which comprise the steps of:

(A) providing the following ingredients:

(a) a medicament present in an amount from about 1% to about 90%, by weight of the microcapsule composition;

(b) a film forming polymer present in an amount from about 8% to about 90%, by weight of the microcapsule composition; and

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture; and

(C) feeding the mixture of step (B) into a spray dryer and spray drying the mixture under controlled conditions such that spheroidal microcapsules under about 150 microns in diameter are formed.

The concentration of the aqueous mixture of medicament in step (B) is sufficiently dilute to prevent coagulation of the medicament. In a preferred embodiment, the concentration of the medicament in step (B) is from about 0.25% to about 50%, preferably from about 1% to about 35%, and most preferably from about 3% to about 25%, by weight of the aqueous mixture.

The concentration of the aqueous mixture of film forming polymer in step (B) is sufficiently dilute to prevent coagulation of the film forming polymer. In a preferred embodiment, the concentration of the film forming polymer in step (B) is from about 5% to about 40%, preferably from about 10% to about 35%, and most preferably from about 10% to about 30%, by weight of the aqueous mixture.

The aqueous homogeneous mixture of step (B) in the method of the present invention is spray dried in step (C) under controlled conditions such that the mixture is flash dried and spheroidal microcapsules of under about 150 microns in diameter are formed. Flash drying (the rapid evaporation of solvent) involves the optimizing of various parameters in the spray drying process such as the liquid feed rate, the air flow rate, the nozzle setting, the pressure of the system and the air inlet and air outlet temperatures. For example, the air outlet temperature must be sufficiently high so that sufficient heat is available to rapidly dry the spray and form small spheroidal microcapsules. The air outlet temperature is dependent upon such other parameters as the air inlet temperature and the liquid feed rate. The aqueous mixture of the present invention may be flash dried using standard techniques and equipment known to those skilled in the art. The exact conditions for flash drying will vary with the particular apparatus selected and are readily determined by those skilled in the art without the need for undue experimentation. Spray drying apparatus is well known in the arts and therefore the selection of the specific apparatus will be apparent to the artisan.

In a preferred embodiment, the spray drying apparatus is a Büchi spray dryer, such as a Büchi 190 Mini Spray dryer. In this embodiment, the liquid feed rate is preferably from about 5 ml/min. to about 25 ml/min., more preferably from about 7 ml/min. to about 22 ml/min., and most preferably from about 9 ml/min. to about 20 ml/min. The air flow rate is preferably from about 300 Nl/h (normliters per hour) to about 750 Nl/h, and more preferably from about 400 Nl/h to about 700 Nl/h, and most preferably from about 500 Nl/h to about 700 Nl/h. The nozzle setting is preferably from about 0.8 mm to about 2.0 mm, more preferably from about 0.8 mm to about 1.5 mm, and most preferably from about 1 mm to abut 1.5 mm. The pressure of the system (system pressure) is preferably from about 25 mm/Hg to about 50 mm/Hg, more preferably from about 30 mm/Hg to about 50 mm/Hg, and most preferably from about 35 mm/Hg to about 50 mm/Hg. The air inlet temperature of the spray dryer is preferably from about 60° C. to about 145° C., more preferably from about 80° C. to about 140° C., and most preferably from about 85° C. to about 135° C. The air outlet temperature of the spray dryer is preferably from about 40° C. to about 90° C., more preferably from about 45° C. to about 90° C., and most preferably from about 50° C. to about 80° C.

The present invention is also directed at a method for preparing the spheroidal coated microcapsules. More particularly, the present invention is directed at a method for preparing spheroidal coated microcapsules under about 850 microns in diameter which comprise at least one spray dried spheroidal microcapsule core under about 150 microns in diameter and a coating layer over the core which comprises (a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5% and (b) a surface active agent present in an amount from about 0.5% to about 20%. The inventive spray dried spheroidal microcapsules may be coated with the edible material by methods well known in the art such that spheroidal coated microcapsules under about 850 microns in diameter are formed. These methods include, for example, the fluidized bed granulation technique, the spray congealing technique, and the pulverization technique.

In one embodiment, the spray dried spheroidal microcapsules are coated by the fluidized bed granulation technique. In this embodiment, the invention is directed at a method for preparing spheroidal coated microcapsules under about 850 microns in diameter which comprise at least one spray dried spheroidal microcapsule core under about 150 microns in diameter and a coating layer over the core, which comprises the steps of:

(A) providing the following ingredients of the microcapsule core:

(a) a medicament present in an amount from about 1% to about 90%, by weight of the core oomposition;

(b) a film forming polymer present in an amount from about 8% to about 90%, by weight of the core composition; and

(c) a plasticizing agent in an amount from about 5% to about 30%, by weight of the film forming polymer; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) in a fluid bed dryer by suspending the microcapsules in a stream of air passing through a zone of the coating layer mixture under controlled conditions such that spheroidal spray coated microcapsules under about 850 microns in diameter are formed.

The spheroidal microcapsules from step (C) in the present invention are spray (film) coated in step (F) in a fluid bed dryer by suspending the microcapsules in a stream of air or strong upward air current and passing the stream through a zone of finely atomized droplets of the coating layer mixture (encapsulant), after which the coated particles pass out of the upward stream and pass downward in a fluidized condition countercurrent to a flow of heated fluidized gas whereupon they are dried, and may re-enter the upward-moving coating zone for a subsequent discreet coating application. The microcapsules of the present invention are spray coated under controlled conditions such that spheroidal coated microcapsules under about 850 microns in diameter are formed. The microcapsules of the present invention may be spray coated using standard techniques and equipment known to those skilled in the art. The exact conditions for spray coating will vary with the particular fluid bed apparatus selected and are readily determined by those skilled in the art without the need for undue experimentation. Fluid bed apparatus is well known in the arts and therefore the selection of the specific apparatus will be apparent to the artisan.

In one preferred embodiment, the method and apparatus employed is known as the Wurster Process. The Wurster Process and its associated apparatus are disclosed in details, for example, in United States patents no. 3,089,824, 3,117,027, 3,196,827, 3,241,520, and 3,253,944, which disclosures are incorporated herein by reference.

In this preferred embodiment, the apparatus is a Versa-Glatt model GPCG 1 fluidized bed apparatus with a Wurster column. In this embodiment, the spray rate during the coating in step (F) is preferably from about 2 ml/min. to about 10 ml/min., more preferably from about 2 ml/min. to about 7 ml/min, and most preferably from about 2 ml/min. to about 6 ml/min. The fluidizing (atomizing) air pressure in step (F) is preferably from about 1 atmosphere to about 5 atmospheres, more preferably from about 1 atmosphere to about 4.5 atmospheres, and most preferably from about 1 atmosphere to abut 4 atmospheres. The nozzle setting in step (F) is preferably from about 0.8 mm to about 2 mm, more preferably from about 0.8 mm to about 1.5 mm, and most preferably from about 0.8 mm to about 1.2 mm. The inlet temperature in step (F) is preferably from about 35° C. to about 65° C., more preferably from about 35° C. to about 60° C., and most preferably from about 40° C. to about 55° C. The outlet temperature in step (F) is preferably from about 20° C. to about 50° C., more preferably from about 25° C. to about 50° C., and most preferably from about 30° C. to about 50° C.

In another preferred embodiment, the spray dried spheroidal microcapsules are coated by the spray congealing technique. In this embodiment, the invention is directed at a method for preparing spheroidal coated microcapsules under about 850 microns in diameter which comprise at least one spray dried spheroidal microcapsule core under about 150 microns in diameter and a coating layer over the core, which comprises the steps of:

(A) providing the following ingredients of the microcapsule core:

(a) a medicament present in an amount from ·about 1% to about 90%, by weight of the core composition;

(b) a film forming polymer present in an amount from about 8% to about 90%, by weight of the core composition;

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, feeding the dispersion into a heat controlled pressure spray nozzle, atomizing the dispersion under controlled conditions such that spheroidal spray congealed coated microcapsules under about 850 microns in diameter are formed.

The coating layer is prepared by mixing at low shear the fatty acid or wax edible material with the surface active agent at temperatures from about 75° C. to about 95° C. until a homogeneous mixture is obtained. The microcapsule core material is then added to this mixture and mixed to uniformly disperse the material in the mixture. The dispersion is then fed into a heat controlled spray nozzle and spray congealed. The term spray congealing as used herein refers to the solidification of the atomized liquid droplets which cool and solidify upon contacting the cooler temperature of the surrounding atmosphere. The nozzle pressure is regulated to control the particle droplet size. The droplets cool and congeal once they are emitted from the nozzle and contact the cooler environment. The resulting dry particles or agglomerates having an approximate elliptical or spherical (spheroidal) shape.

The microcapsules of the present invention are spray congealed under controlled conditions such that spheroidal coated microcapsules under about 850 microns in diameter are formed. The microcapsules of the present invention may be spray congealed using standard techniques and equipment known to those skilled in the art. The exact conditions for spray congealing will vary with the particular apparatus selected and are readily determined by those skilled in the art without the need for undue experimentation. Spray congealing apparatus is well known in the arts and therefore the selection of the specific apparatus will be apparent to the artisan. The coated microcapsules may then be screened to the desired mesh size.

In an alternative, but less preferred embodiment, the spray dried spheroidal microcapsules are coated by the pulverization technique. In this embodiment, the invention is directed at a method for preparing spheroidal coated microcapsules under about 850 microns in diameter which comprise at least one spray dried spheroidal microcapsule core under about 150 microns in diameter and a coating layer over the core, which comprises the steps of:

(A) providing the following ingredients of the microcapsule core:

(a) a medicament present in an amount from about 1% to about 90%, by weight of the core composition;

(b) a film forming polymer present in an amount from about 8% to about 90%, by weight of the core composition; and

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, feeding the dispersion to cool, and pulverizing the cooled dispersion under controlled conditions such that spheroidal coated microcapsules under about 850 microns in diameter are formed.

The uniform mixture of spray dried microcapsules and coating layer mixture of edible material and surface active agent are cooled in sheets and pulverized to a particle size from about 75 microns to about 850 microns, preferably from about 75 microns to about 750 microns, and more preferably from about 75 microns to about 650 microns.

In another embodiment, the present invention is directed at a spheroidal coated microcapsule under about 850 microns in diameter which comprises at least one spray dried spheroidal microcapsule core under about 150 microns in diameter and a coating layer over the core, prepared by a method which comprises the steps of:

(A) providing the following ingredients of the microcapsule core:

(a) a medicament present in an amount from about 1% to about 90%, by weight of the core composition;

(b) a film forming polymer present in an amount from about 8% to about 90%, by weight of the core composition;

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) in a fluid bed dryer by suspending the microcapsules in a stream of air passing through a zone of the coating layer mixture under controlled conditions such that spheroidal spray coated microcapsules under about 850 microns in diameter are formed.

In another embodiment, the present invention is directed at a spheroidal coated microcapsule under about 850 microns in diameter which comprises at least one spray dried spheroidal microcapsule core under about 150 microns in diameter and a coating layer over the core, prepared by a method which comprises the steps of:

(A) providing the following ingredients of the microcapsule core:

11

(a) a medicament present in an amount from about 1% to about 90%, by weight of the core composition;

(b) a film forming polymer present in an amount from about 8% to about 90%, by weight of the core composition;

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, feeding the dispersion into a heat controlled pressure spray nozzle, atomizing the dispersion under controlled conditions such that spheroidal spray congealed coated microcapsules under about 850 microns in diameter are formed.

In another embodiment, the present invention is directed at a spheroidal coated microcapsule under about 850 microns in diameter which comprises at least one spray dried spheroidal microcapsule core under about 150 microns in diameter and a coating layer over the core, prepared by a method which comprises the steps of:

(A) providing the following ingredients of the microcapsule core:

(a) a medicament present in an amount from about 1% to about 90%, by weight of the core composition;

(b) a film forming polymer present in an amount from about 8% to about 90%, by weight of the core composition;

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, allowing the dispersion to cool, and pulverizing the cooled dispersion under controlled conditions such that spheroidal coated microcapsules under about 850 microns in diameter are formed.

Once prepared, the chewable spray dried spheroidal microcapsule and coated microcapsule compositions may be stored for future use or may be formulated with conventional additives such as pharmaceutically acceptable carriers and confectionery bulking agents to prepare a wide variety of chewable medicated compositions and sustained release compositions to suit particular applications.

An important aspect of the present invention includes a hard or soft confectionery composition incorporating the inventive spray dried spheroidal microcapsules and coated microcapsule compositions and a method for preparing the hard or soft confections. In this form of the invention, the spray dried spheroidal microcapsules and coated microcapsules include a pharmaceutically acceptable carrier such as a confectionery bulking agent, the inventive chewable microcapsule compositions, and various additives. The confectionery may be in the form of a lozenge, tablet, toffee, nougat, suspension, chewy candy, and the like. The pharmaceutically acceptable carriers may be prepared from a wide range of materials. Without being limited thereto, such materials include diluents, binders and adhesives, lubricants, disintegrants, coloring agents, bulking agents, flavoring agents, sweetening agents and miscellaneous materials such as buffers and adsorbents in order to prepare a particular medicated chewable confection.

The preparation of confectionery formulations is historically well known and has changed little through the years. Confectionery items have been classified as either "hard" confectionery or "soft" confectionery. The chewable medicated compositions of the present invention can be incorporated into confectionery compositions by admixing the inventive compositions into conventional hard and soft confections.

As used herein, the term confectionery material means a product containing a bulking agent selected from a wide variety of materials such as sugar, corn syrup, and the like, and in the case of sugarless bulking agents, sugar alcohols such as sorbitol and mannitol and the like, and mixtures thereof. Confectionery material may include such exemplary substances as lozenges, tablets, toffee, nougat, suspensions, chewy candy, chewing gum and the like. The bulking agent is present in a quantity sufficient to bring the total amount of confectionery composition to 100%.

Lozenges are flavored medicated dosage forms intended to be sucked and held in the mouth. Lozenges may be in the form of various shapes such as flat, circular, octagonal and biconvex forms. The lozenge bases are generally in two forms: hard, boiled candy lozenges and compressed tablet lozenges.

Hard boiled candy lozenges may be processed and formulated by conventional means. In general, a hard boiled candy lozenge has a base composed of a mixture of sugar and other carbohydrate bulking agents kept in an amorphous or glassy condition. This amorphous or glassy form is considered a solid syrup of sugars generally having from about 0.5% to about 1.5% moisture. Such materials normally contain up to about 92% corn syrup, up to about 55% sugar and from about 0.1% to about 5% water, by weight of the final composition. The syrup component is generally prepared from corn syrups high in fructose, but may include other materials. Further ingredients such as flavoring agents, sweetening agents, acidulants, coloring agents and the like may also be added.

Boiled candy lozenges may also be prepared from non-fermentable sugars such as sorbitol, mannitol, and hydrogenated corn syrup. Typical hydrogenated corn syrups are Lycasin, a commercially available product manufactured by Roquette Corporation, and Hystar, a commercially available product manufactured by Lonza, Inc. The candy lozenges may contain up to about 95% sorbitol, a mixture of sorbitol and mannitol in a ratio from about 9.5:0.5 up to about 7.5:2.5, and hydrogenated corn syrup up to about 55%, by weight of the solid syrup component.

Boiled candy lozenges may be routinely prepared by conventional methods such as those involving fire cookers, vacuum cookers, and scraped-surface cookers also referred to as high speed atmospheric cookers.

Fire cookers involve the traditional method of making a boiled candy lozenge base. In this method, the desired quantity of carbohydrate bulking agent is dissolved in water by heating the agent in a kettle until the bulking agent dissolves. Additional bulking agent may then be added and the cooking continued until a final temperature of 145° C. to 156° C. is achieved. The batch is then cooled and worked as a plastic-like mass to incorporate additives such as flavoring agents, coloring agents and the like.

A high-speed atmospheric cooker uses a heat-exchanger surface which involves spreading a film of candy on a heat exchange surface, the candy is heated to 165° C. to 170° C. in a few minutes. The candy is then rapidly cooled to 100° C. to 120° C. and worked as a plastic-like mass enabling incorporation of the additives, such as flavor agents, coloring agents and the like.

In vacuum cookers, the carbohydrate bulking agent is boiled at a temperature from about 125° C. to about 132° C., vacuum is applied and additional water is boiled off without extra heating. When cooking is complete, the mass is a semi-solid and has a plastic-like consistency. At this point, flavoring agents, coloring agents, and other additives are admixed in the mass by routine mechanical mixing operations.

The optimum mixing required to uniformly mix the flavoring agents, coloring agents and other additives during conventional manufacturing of boiled candy lozenges is determined by the time needed to obtain a uniform distribution of the materials. Normally, mixing times of from about 4 to about 10 minutes have been found to be acceptable.

Once the boiled candy lozenge has been properly tempered, it may be cut into workable portions or formed into desired shapes. A variety of forming techniques may be utilized depending upon the shape and size of the final product desired. A general discussion of the composition and preparation of hard

EP 0 421 581 A1

confections may be found in H.A. Lieberman, Pharmaceutical Dosage Forms: Tablets, Volume 1 (1980), Marcel Dekker, Inc., New York, N.Y. at pages 339 to 469, which disclosure is incorporated herein by reference.

The apparatus useful in accordance with the present invention comprises cooking and mixing apparatus well known in the confectionery manufacturing arts, and therefore the selection of the specific apparatus will be apparent to the artisan.

In contrast, compressed tablet confections contain particulate materials and are formed into structures under pressure. These confections generally contain sugars in amounts up to about 95%, by weight of the composition, and typical tablet excipients such as binders and lubricants as well as flavoring agents, coloring agents and the like.

In addition to hard confectionery materials, the lozenges of the present invention may be made of soft confectionery materials such as those contained in nougat. The preparation of soft confections, such as nougat, involves conventional methods, such as the combination of two primary components, namely (1) a high boiling syrup such as a corn syrup, hydrogenated starch hydrolysate or the like, and (2) a relatively light textured frappe, generally prepared from egg albumin, gelatin, vegetable proteins, such as soy derived compounds, sugarless milk derived compounds such as milk proteins, and mixtures thereof. The frappe is generally relatively light, and may, for example, range in density from about 0.5 to about 0.7 grams/cc.

The high boiling syrup, or "bob syrup" of the soft confectionery is relatively viscous and has a higher density than the frappe component, and frequently contains a substantial amount of carbohydrate bulking agent such as a hydrogenated starch hydrolysate. Conventionally, the final nougat composition is prepared by the addition of the "bob syrup" to the frappe under agitation, to form the basic nougat mixture. Further ingredients such as flavoring agents, additional carbohydrate bulking agents, colouring agents, preservatives, medicaments, mixtures thereof and the like may be added thereafter also under agitation. A general discussion of the composition and preparation of nougat confections may be found in B.W. Minifie, Chocolate, Cocoa and Confectionery: Science and Technology, 2nd edition, AVI Publishing Co., Inc., Westport, Conn. (1980), at pages 424-425, which disclosure is incorporated herein by reference.

The procedure for preparing the soft confectionery involves known procedures. In general, the frappe component is prepared first and thereafter the syrup component is slowly added under agitation at a temperature of at least about 65o C., and preferably at least about 100o C. The mixture of components is continued to be mixed to form a uniform mixture, after which the mixture is cooled to a temperature below 80o C., at which point, the flavoring agent may be added. The mixture is further mixed for an additional period until it is ready to be removed and formed into suitable confectionery shapes.

The novel chewable medicated microcapsules and sustained release compositions may also be in the form of a pharmaceutical suspension. Pharmaceutical suspensions of this invention may be prepared by conventional methods long established in the art of pharmaceutical compounds. Suspensions may contain adjunct materials employed in formulating the suspensions of the art. The suspensions of the present invention can comprise:

(a) preservatives such as benzoic acid, sorbic acid, methyl paraben, and propyl paraben. Preservatives are generally present in amounts up to about 1%, and preferably from about 0.05% to about 0.5%, by weight of the suspension;

(b) buffers such as citric acid-sodium citrate, phosphoric acid-sodium phosphate, and acetic acid-sodium acetate which may be present in amounts up to about 1%, and preferably from about 0.05% to about 0.5%, by weight of the suspension;

(c) suspending agents or thickeners such as cellulosics like methylcellulose, carrageenans like alginic acid and its derivatives, xanthan gums, gelatin, acacis, and microcrystalline cellulose which may be present in amounts up to about 20%, and preferably from about 1% to about 15%, by weight of the suspension;

(d) antifoaming agents such as dimethyl polysiloxane which may be present in amounts up to about 0.2%, and preferably from about 0.01% to about 0.1%, by weight of the suspension;

(e) sweetening agents such as those sweeteners well known in the art, including both natural and artificial sweeteners. Sweetening agents such as monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, glycyrrhizin, and sugar alcohols such as sorbitol, mannitol, maltitol, hydrogenated starch hydrolysates and mixtures thereof may be utilized in amounts up to about 60%, and preferably from about 20% to about 50%, by weight of the suspension. Water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide,

14

the potassium salt of 3,4-dihydro-6-methyl-1,2,3-oxathiazine-4-one-2,2-dioxide (Acesulfame-K), the free acid form of saccharin, and the like may be utilized in amounts from about 0.001% to about 5%, by weight of the suspension;

(f) flavoring agents such as those flavors well known to the skilled artisan, such as natural and artificial flavors and mints, such as peppermint, menthol, citrus flavors such as orange and lemon, artificial vanilla, cinnamon, various fruit flavors, both individual and mixed and the like may be utilized in amounts from about 0.5% to about 5%, by weight of the suspension;

(g) coloring agents such as pigments which may be incorporated in amounts up to about 6%, by weight of the suspension. A preferred pigment, titanium dioxide, may be incorporated in amounts up to about 2%, and preferably less than about 1%, by weight of the suspension. The coloring agents may also include natural food colors and dyes suitable for food, drug and cosmetic applications. These coloring agents are known as F.D.& C. dyes and lakes. The materials acceptable for the foregoing uses are preferably water-soluble. Such dyes are generally present in amounts up to about 0.25%, and preferably from about 0.05% to about 0.2%, by weight of the suspension;

(h) decolorizing agents such as sodium metabisulfite, ascorbic acid and the like may be incorporated into the suspension to prevent color changes due to aging. In general, decolorizing agents may be used in amounts up to about 0.25%, and preferably from about 0.05% to about 0.2%, by weight of the suspension; and

(i) vehicles such as propylene glycol, polyethylene glycol, edible oils such as animal, vegetable and mineral oils, and the like may be used to solubilize the flavoring agents. In general, vehicles may be used in amounts up to about 10%, and preferably from about 2% to about 5%, by weight of the suspension.

The pharmaceutical suspensions of the present invention may be prepared as follows:

(A) admix the thickener with the vehicle heated to a temperature from about 40° C. to about 95° C., preferably from about 40° C. to about 70° C., to form a dispersion if the thickener is soluble in the vehicle or a solution if the thickener is soluble in the soluble;

(B) admix the sweetening agent with the vehicle to form a solution;

(C) admix the sustained release composition with the thickener-vehicle admixture to form a uniform thickener-sustained release composition;

(D) combine the sweetener solution with the thickener-sustained release composition and mix until uniform; and

(E) admix the optional adjunct materials such as coloring agents, flavoring agents, decolorants, solubilizing agents, antifoaming agents, buffers and additional vehicle with the mixture of step (D) to form the suspension.

To achieve acceptable stability and quality as well as good taste and mouth feel in a chewable sustained release formulation several considerations are important. These considerations include the amount of active substance per tablet, the flavoring agent employed, the degree of compressibility of the tablet and the organoleptic properties of the pharmaceutical composition.

Chewable medicated candy is prepared by procedures similar to those used to make soft confectionery. In a typical procedure, a boiled sugar-corn syrup blend is formed to which is added a frappe mixture. The boiled sugar-corn syrup blend may be prepared from sugar and corn syrup blended in parts by weight ratio of about 90:10 to about 10:90. The sugar-corn syrup blend is heated to temperatures above about 120° C. to remove water and to form a molten mass. The frappe is generally prepared from gelatin, egg albumin, milk proteins such as casein, and vegetable proteins such as soy protein, and the like, which is added to a gelatin solution and rapidly mixed at ambient temperature to form an aerated sponge like mass. The frappe is then added to the molten candy mass and mixed until homogeneous at temperatures between about 65° C. and about 120° C.

The spray dried spheroidal microcapsules and sustained release compositions of the instant invention can then be added to the homogeneous mixture as the temperature is lowered to about 65° C.-95° C. whereupon additional ingredients can then be added such as flavoring agents and coloring agents. The formulation is further cooled and formed into pieces of desired dimensions.

A general discussion of the lozenge and chewable tablet forms of confectionery may be found in H.A. Lieberman and L. Lachman, Pharmaceutical Dosage Forms: Tablets Volume 1, Marcel Dekker, Inc., New York, N.Y. at pages 289 to 466, which disclosure is incorporated herein by reference.

In accordance with this invention, therapeutically effective amounts of the chewable microcapsule and coated microcapsule compositions of the present invention may be admixed into the hard and soft confections. These amounts are readily determined by those skilled in the art without the need for undue experimentation. The exact amount of the spray dried spheroidal microcapsules and coated microcapsules employed in the hard and soft confections will vary with the particular medicament selected. In a preferred embodiment, the spray dried spheroidal microcapsules and coated microcapsules are present in the hard and soft confection compositions in percentages by weight in an amount from about 5% to about 50%, more preferably from about 10% to about 40%, and most preferably, in an amount from about 10% to about 30%. The pharmaceutically acceptable carrier and optional additives are present in a quantity sufficient to bring the total amount of hard and soft confection composition to 100%.

The present invention extends to methods of making the improved chewable medicated hard and soft confection compositions. The medicated microcapsule compositions may be incorporated into otherwise conventional or soft confection compositions using standard techniques and equipment known to those skilled in the art.

The apparatus useful in accordance with the present invention comprises cooking and mixing apparatus well known in the confectionery manufacturing arts, and therefore the selection of the specific apparatus will be apparent to the artisan.

In one embodiment, the present invention is directed at a chewable medicated composition which comprises a pharmaceutically acceptable carrier and a therapeutically effective amount of spray dried spheroidal microcapsules under about 150 microns in diameter, wherein the microcapsules comprise:

(a) a medicament present in an amount from about 1% to about 90%, by weight of the microcapsule composition;

(b) a film forming polymer present in an amount from about 8% to about 90%, by weight of the microcapsule composition; and

(c) a plasticizing agent in an amount from about 5% to about 30% by weight of the film forming agent.

In another embodiment, the present invention is directed at a chewable sustained release medicated composition which comprises a pharmaceutically acceptable carrier and a therapeutically effective amount of spheroidal coated microcapsules under about 850 microns in diameter which comprise a least one spray dried spheroidal microcapsule core under about 150 microns in diameter and a coating layer over the core, wherein the coated microcapsules comprise:

(A) a microcapsule core comprising:

(a) a medicament present in an amount from about 1% to about 90%, by weight of the core composition;

(b) a film forming polymer present in an amount from about 8% to about 90%, by weight of the core composition; and

(c) a plasticizing agent in an amount from about 5% to about 30%, by weight of the film forming agent; and

(B) a coating layer over the core comprising in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%.

In another embodiment, the present invention is directed at a chewable medicated composition which comprises a pharmaceutically acceptable carrier and a therapeutically effective amount of spray dried spheroidal microcapsules under about 150 microns in diameter, wherein the microcapsules are prepared by a method which comprises the steps of:

(A) providing the following ingredients:

(a) a medicament present in an amount from about 1% to about 90%, by weight of the microcapsule composition;

(b) a film forming polymer present in an amount from about 8% to about 90%, by weight of the microcapsule composition; and

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture; and

(C) feeding the mixture of step (B) into a spray dryer and spray drying the mixture under controlled conditions such that spheroidal microcapsules under about 150 microns in diameter are formed.

In another embodiment, the present invention is directed at a chewable sustained release medicated composition which comprises a pharmaceutically acceptable carrier and a therapeutically effective amount of spheroidal coated microcapsules under about 850 microns in diameter which comprise at least one spray dried spheroidal microcapsule core under about 150 microns in diameter and a coating layer over the core, wherein the coated microcapsules are prepared by a method which comprises the steps of:

(A) providing the following ingredients of the microcapsule core:

(a) a medicament present in an amount from about 1% to about 90%, by weight of the core composition;

(b) a film forming polymer present in an amount from about 8% to about 90%, by weight of the core composition; and

(c) a plasticizing agent in an amount from about 5% to about 30%, by weight of the film forming polymer; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) in a fluid bed dryer by suspending the microcapsules in a stream of air passing through a zone of the coating layer mixture under controlled conditions such that spheroidal spray coated microcapsules under about 850 microns in diameter are formed.

In another embodiment, the present invention is directed at a chewable sustained release medicated composition which comprises a pharmaceutically acceptable carrier and a therapeutically effective amount of spheroidal coated microcapsules under about 850 microns in diameter which comprise at least one spray dried spheroidal microcapsule core under about 150 microns in diameter and a coating layer over the core, wherein the coated microcapsules are prepared by a method which comprises the steps of:

(A) providing the following ingredients of the microcapsule core:

(a) a medicament present in an amount from about 1% to about 90%, by weight of the core composition;

(b) a film forming polymer present in an amount from about 8% to about 90%, by weight of the core composition; and

(c) a plasticizing agent in an amount from about 5% to about 30%, by weight of the film forming agent; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, feeding the dispersion into a heat controlled pressure spray nozzle, atomizing the dispersion under controlled conditions such that spheroidal spray congealed coated microcapsules under about 850 microns in diameter are formed.

In another embodiment, the present invention is directed at a chewable sustained release medicated composition which comprises a pharmaceutically acceptable carrier and a therapeutically effective amount of spheroidal coated microcapsules under about 850 microns in diameter which comprise at least one spray dried spheroidal microcapsule core under about 150 microns in diameter and a coating layer over the core, wherein the coated microcapsules are prepared by a method which comprises the steps of:

(A) providing the following ingredients of the microcapsule core:

(a) a medicament present in an amount from about 1% to about 90%, by weight of the core composition;

(b) a film forming polymer present in an amount from about 8% to about 90%, by weight of the core composition; and

(c) a plasticizing agent in an amount from about 5% to about 30%, by weight of the film forming polymer; and

(B) preparing an aqueous homogeneous mixture of the ingredients in step (A), wherein the medicament is present in a concentration from about 0.25% to about 50%, and the film forming polymer is present in a concentration from about 5% to about 40%, by weight of the aqueous mixture;

(C) feeding the mixture of step (B) into a spray dryer and spray drying the mixture under controlled conditions such that spheroidal microcapsules under about 150 microns in diameter are formed;

(D) providing the following ingredients of the coating layer, in percentages by weight of the coating layer composition:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 80% to about 99.5%; and

(b) a surface active agent present in an amount from about 0.5% to about 20%; and

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, allowing the dispersion to cool, and pulverizing the cooled dispersion under controlled conditions such that spheroidal coated microcapsules under about 850 microns in diameter are formed.

The present invention is further illustrated by the following examples which are not intended to limit the effective scope of the claims. All parts and percentages in the examples and throughout the specification and claims are by weight of the final composition unless otherwise specified.

EXAMPLE 1

This Example demonstrates a method for preparing spray dried spheroidal microcapsules and coated microcapsules by the pulverizing technique according to the process of the present invention.

A spray dried spheroidal microcapsule or core particle was prepared having the composition set out in Table 1.

TABLE 1

| SPRAY DRIED MICROCAPSULE COMPOSITION | | | |
|---|---|---|---|
| INGREDIENT | AMOUNT | % BY WEIGHT MICROCAPSULE | % BY WEIGHT AQUEOUS MIXTURE |
| Pseudoephedrine sulfate | 75g | 22.56 | 3.60 |
| Aluminum stearate | 7.5g | 2.26 | 0.36 |
| Ethyl Cellulose [1000g of 25% aqueous SURELEASE] | 250g | 75.19 | 12.00 |
| 1% Ammonium Hydroxide | 1000g | | |

Pseudoephedrine sulfate and aluminum stearate were slurried in the aqueous suspension of ethyl cellulose and plasticizing agent and mixed at about 30 rpm to form a uniform dispersion. The resulting mixture was spray dried in a Büchi 190 Mini Spray Dryer wherein the liquid feed rate was 10 ml/minute, the air flow rate was 500 Nl/h, the nozzle setting was 1.2 mm, the system pressure was 30 mm/Hg, the air inlet temperature was from about 115° C. to about 90° C., and the air outlet temperature was from about 60° C. to about 66° C. A quantity of 300 g of the spray dried microcapsules containing pseudoephedrine sulfate were obtained which were passed through a 100 mesh screen (greater than 90% of the microcapsules passed through the screen).

A quantity of 150g of the pseudoephedrine sulfate microcapsule core material prepared above was coated with a coating layer material which had the composition set out in Table 2.

TABLE 2

| COATING LAYER COMPOSITION | | |
|---|---|---|
| INGREDIENT | AMOUNT | PERCENTAGE BY WEIGHT |
| Carnauba wax | 267g | 89 |
| Bees wax | 30g | 10 |
| Stearic acid | 3g | 1 |

The coating layer material was loaded into a steam heated Hobart mixer at a temperature of around 92° C. and mixed until homogeneous. The pseudoephedrine sulfate microcapsule core material prepared above was then admixed with the coating layer mixture and stirred at about 70 rpm until the mixture was homogeneous. The coated microcapsule mixture was allowed to cool to room temperature and then was pulverized into particles which were separated into the following mesh sizes: 30-60, 60-120, and 120-200. The coated microcapsules contained 7.52% pseudoephedrine sulfate.

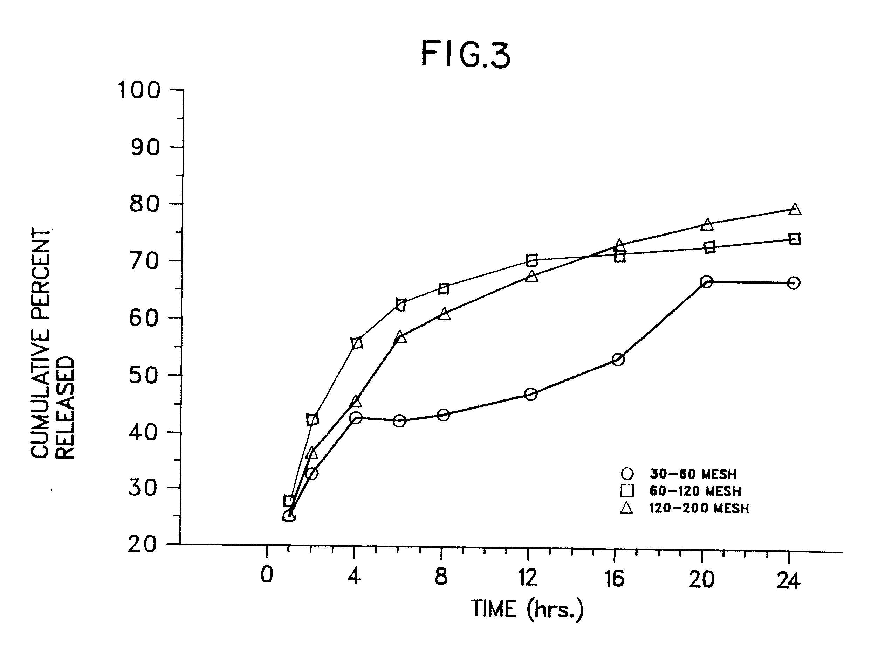

The sustained release properties (dissolution) of the coated microcapsules having mesh size 30-60 (sample 1), mesh size 60-120 (sample 2) and mesh size 120-200 (sample 3) in nougat composition were measured by the paddle method (The United States Pharmacopeia XXI) at 100 rpm at 37° C. Each nougat was cut into 32 pieces and then introduced into the dissolution apparatus with 1000ml of distilled water. The dissolution of the medicament from the coated microcapsule samples was measured by High Pressure Liquid Chromatography using an ultraviolet detector. The results of the dissolution study are set out in FIGURE 3.

FIGURE 3 shows that the sustained release properties for pulverized coated microcapsules having a mesh size from about 60 to about 200 mesh are similar. FIGURE 3 also shows that the dissolution rate of the microcapsules having a mesh size larger than about 60 mesh is similar to the microcapsules having a mesh size from about 60 to about 200 mesh during the first four hours and much slower from four hours to twenty four hours.

EXAMPLE 2

This Example demonstrates a method for preparing spray dried spheroidal microcapsules and coated microcapsules by the fluid bed granulation technique according to the process of the present invention.

A spray dried spheroidal microcapsule or core particle was prepared having the composition set out in Table 3.

TABLE 3

| SPRAY DRIED MICROCAPSULE COMPOSITION | | | |
|---|---|---|---|
| INGREDIENT | AMOUNT | % BY WEIGHT MICROCAPSULE | % BY WEIGHT AQUEOUS MIXTURE |
| Pseudoephedrine sulfate | 100g | 16.13 | 2.70 |
| Aluminum stearate | 20g | 3.22 | 0.54 |
| Ethyl Cellulose [2000g of 25% aqueous suspension of SURELEASE] | 500g | 80.65 | 13.51 |
| 1% Ammonium Hydroxide | 1580g | | |

EP 0 421 581 A1

A homogeneous mixture of the above ingredients was prepared essentially according to the procedure of Example 1. The resulting mixture was spray dried in a Büchi 190 Mini Spray Dryer wherein the liquid feed rate was 10 ml/minute, the air flow rate was 500 Nl/h, the nozzle setting was 1.2 mm, the system pressure was 30 mm/Hg, the air inlet temperature was from about 115° C. to about 90° C., and the air outlet temperature was from about 60° C. to about 66° C. A quantity of 560g of the spray dried microcapsules containing pseudoephedrine sulfate were obtained which were passed through a 100 mesh screen (greater than about 90% of the microcapsules passed through the screen).

A quantity of 340g of the spray dried microcapsule core material containing pseudoephedrine sulfate prepared above was coated with a coating material which had the composition set out in Table 4.

TABLE 4

| COATING LAYER COMPOSITION | | |
|---|---|---|
| INGREDIENT | AMOUNT | PERCENTAGE BY WEIGHT |
| Carnauba wax | 525g | 52.5 |
| Glyceryl tristearate | 400g | 40.0 |
| Paraffin wax 150 | 50g | 5.0 |
| Dimodan PVPK | 25g | 2.5 |

The coating layer material was loaded into a steam heated Hobart mixer and mixed until homogeneous. The pseudoephedrine sulfate microcapsule core material prepared above was fluidized in a Versa-Glatt model GPCG 1 fluidized bed apparatus. The fluidized bed apparatus was then heated to the spraying-processing temperature. The pseudoephedrine sulfate microcapsules were then spray coated by heated air atomization with the coating layer mixture until a 100% weight gain was obtained. The coated microcapsules were then removed from the apparatus. A quantity of 972g of the film coated microcapsules were obtained which contained 5.64% pseudoephedrine sulfate (sample 3).

The sustained release properties (dissolution) of the coated microcapsules having mesh size 40-80 in nougat composition, prepared by conventional methods, were measured by the paddle method (The United States Pharmacopeia XXI) at 100 rpm at 37° C. Each nougat was cut into 32 pieces and then introduced into the dissolution apparatus with 1000ml of distilled water. The dissolution of the medicament from the coated microcapsule samples was measured by High Pressure Liquid Chromatography using an ultraviolet detector. The results of the dissolution study are set out in Table 5.

TABLE 5

| TIME (hours) | SAMPLE 3 (% Dissolution by Weight) |
|---|---|
| 1 | 29.31 |
| 2 | 44.28 |
| 4 | 62.52 |
| 6 | 74.39 |
| 8 | 80.22 |
| 12 | 86.09 |
| 16 | 96.62 |

Table 5 shows satisfactory sustained release properties for the spray coated microcapsules of Sample 3.

EXAMPLE 3

This Example demonstrates a method for preparing spray dried spheroidal microcapsules and coated microcapsules by the spray congealing technique according to the process of the present invention.

A spray dried spheroidal microcapsule or core particle was prepared having the composition set out in Table 6.

TABLE 6

| SPRAY DRIED MICROCAPSULE COMPOSITION | | | |
|---|---|---|---|
| INGREDIENT | AMOUNT | % BY WEIGHT MICROCAPSULE | % BY WEIGHT AQUEOUS MIXTURE |
| Pseudoephedrine sulfate | 75g | 22.01 | 3.53 |
| Aluminum stearate | 15g | 4.41 | 0.71 |
| Ethyl Cellulose [1000g of 25% aqueous SURELEASE] | 250g | 75.53 | 11.77 |
| 1% Ammonium Hydroxide | 1035g | | |

A homogeneous mixture of the above ingredients was prepared essentially according to the procedure of Example 1. The resulting mixture was spray dried in a Büchi 190 Mini Spray Dryer wherein the liquid feed rate was 10 ml/minute, the air flow rate was 500 Nl/h, the nozzle setting was 1.2 mm, the system pressure was 30 mm/Hg, the air inlet temperature was from about 115° C. to about 90° C., and the air outlet temperature was from about 60° C. to about 66° C. A quantity of 305g of the spray dried microcapsules containing pseudoephedrine sulfate were obtained which were passed through a 100 mesh screen (greater than about 90% of the microcapsules passed through the screen).

A quantity of 150g of the spray dried microcapsule core material containing pseudoephedrine sulfate prepared above was coated with a coating material which had the composition set out in Table 7.

TABLE 7

| COATING LAYER COMPOSITION | | |
|---|---|---|
| INGREDIENT | AMOUNT | PERCENTAGE BY WEIGHT |
| Glyceryl tristearate | 405g | 90.0 |
| Glyceryl monostearate | 22.5g | 5.0 |
| Cetodan | 22.5g | 5.0 |

The coating layer material was loaded into a steam heated Hobart mixer, then mixed at temperatures from about 75° C. to about 95° C. until a homogeneous mixture was obtained. The pseudoephedrine sulfate microcapsule core material prepared above was then added to this coating layer mixture and mixed at high shear to uniformly disperse the microcapsules in the mixture. The dispersion was then fed into a heat controlled spray nozzle and spray congealed. The nozzle pressure was regulated to control the particle droplet size. A quantity of 600g of the coated microcapsules was obtained which contained 5.5% pseudoephedrine sulfate.

The sustained release properties (dissolution) of the coated microcapsules having mesh size 40-60 (sample 4) and mesh size 60-80 (sample 5) in nougat composition were measured by the paddle method (The United States Pharmacopeia XXI) at 100 rpm at 37° C. Each nougat was cut into 32 pieces and then introduced into the dissolution apparatus with 1000ml of distilled water. The dissolution of the medicament from the coated microcapsule samples was measured by High Pressure Liquid Chromatography using an ultraviolet detector. The results of the dissolution study are set out in Table 8.

TABLE 8

| TIME (hours) | SAMPLE 4 | 5 |
|---|---|---|
| | (% Dissolution by Weight) | |
| 1 | 36.32 | 40.95 |
| 2 | 49.94 | 63.25 |
| 4 | 64.09 | 80.23 |
| 6 | 73.20 | 86.69 |
| 8 | 84.99 | 86.23 |
| 12 | 89.71 | 91.13 |
| 16 | 93.99 | 94.61 |
| 24 | 99.52 | 91.02 |

Table 8 shows that the dissolution rate for spray congeal coated microcapsules having a mesh size from about 40 to about 60 mesh (Sample 4) is better than the dissolution rate for spray congeal coated microcapsules having a mesh size from about 60 to about 80 mesh (Sample 5).

The invention being thus described, it will be obvious that the same may be varied in many ways. Such variations are not to be regarded as a departure from the spirit and scope of the invention and all such modifications are intended to be included within the scope of the following claims.

**Claims**

1. A spheroidal coated microcapsule under about 850 microns in diameter, which comprises at least one spray dried spheroidal microcapsule core under about 150 microns in diameter and a coating layer over the core, wherein (A) the microcapsule comprises:
(a) a medicament present in an amount of from about 1% to about 90%, by weight of the core;
(b) a film forming polymer present in an amount of from about 8% to about 90%, by weight of the core; and
(c) a plasticizing agent in an amount of from about 5% to about 30%, by weight of the film forming polymer; and wherein (B) the coating layer over the core comprises in percentages by weight of the coating layer:
(a) an edible material having a melting point of from about 25°C to about 100°C and selected from (i) fatty acids having an iodine value of from about 1 to about 10, (ii) natural waxes, (iii) synthetic waxes, and (iv) mixtures thereof, present in an amount of from about 80% to about 99.5%; and
(b) a surface active agent present in an amount of from about 0.5% to about 20%.

2. A coated microcapsule according to claim 1, wherein the medicament in the microcapsule core is present in an amount of from about 1% to about 70%, by weight of the core.

3. A coated microcapsule according to claim 1 or 2, wherein the medicament in the microcapsule core is a water-soluble medicament selected from dextromethorphan, dextromethorphan hydrobromide, pseudoephedrine, pseudoephedrine sulfate, pseudoephedrine hydrochloride, pseudoephedrine hydrobromide, chlorpheniramine maleate, guaifenesin, diphenhydramine hydrochloride, and mixtures thereof.

4. A coated microcapsule according to claim 3, wherein the medicament is selected from pseudoephedrine sulfate, diphenhydramine hydrochloride, chlorpheniramine maleate, and mixtures thereof.

5. A coated microcapsule according to any of claims 1 to 4, wherein the film forming polymer in the microcapsule core is present in an amount of from about 20% to about 90%, by weight of the core.

6. A coated microcapsule according to any of claims 1 to 5, wherein the film forming polymer in the microcapsule core is a cellulose derivative selected from cellulose acetate, cellulose acetate butyrate, cellulose acetate phthalate, ethylcellulose, hydroxypropylcellulose, hydroxypropylmethylcellulose, hydroxypropylmethylcellulose phthalate, methylcellulose, sodium carboxymethylcellulose, and mixtures thereof.

7. A coated microcapsule according to claim 6, wherein the film forming polymer in the microcapsule core is ethyl cellulose.

8. A coated microcapsule according to any of claims 1 to 7, wherein the microcapsule core further comprises a dispersing agent present in an amount of up to about 10%, by weight of the core.

EP 0 421 581 A1

9. A coated microcapsule according to claim 8, wherein the dispersing agent in the microcapsule core is selected from aluminum stearate, kaolin, fumed silica, and mixtures thereof.

10. A coated microcapsule according to any of claims 1 to 9, wherein the edible material in the coating layer is present in an amount of from about 85% to about 99.5%, by weight of the coating layer.

11. A coated microcapsule according to any of claims 1 to 10, wherein the edible material in the coating layer is selected from hydrogenated palm oil, hydrogenated castor oil, hydrogenated cottonseed oil, stearic acid, palmitic acid, and mixtures thereof.

12. A coated microcapsule according to claim 11, wherein the edible material in the coating layer is stearic acid.

13. A coated microcapsule according to any of claims 1 to 12, wherein the surface active agent in the coating layer is present in an amount of from about 0.5% to about 15%, by weight of the coating layer.

14. A coated micrccapsule according to any of claims 1 to 13, wherein the surface active agent in the coating layer is selected from glyceryl monostearate, acetylated monoglycerides, stearic acid, and mixtures thereof.

15. A coated microcapsule according to any of claims 1 to 14, wherein the weight ratio of microcapsule core to coating layer is from about 1:9 to about 9:1.

16. A coated microcapsule according to any of claims 1 to 15, wherein the microcapsule is under about 750 microns in diameter.

17. A method for preparing spheroidal coated microcapsules under about 850 microns in diameter which comprise at least one spray dried spheroidal microcapsule core under about 150 microns in diameter and a coating layer over the core, which method comprises the steps of:

(A) providing the following ingredients of the microcapsule core:

(a) a medicament present in an amount of from about 1% to about 90%, by weight of the core;

(b) a film forming polymer present in an amount of from about 8% to about 90%, by weight of the core; and

(c) a plasticizing agent in an amount of from about 5% to about 30%, by weight of the film forming polymer;

(B) preparing an aqueous homogeneous mixture of the ingredients in step (A), wherein the medicament is present in a concentration of from about 0.25% to about 50%, and the film forming polymer is present in a concentration of from about 5% to about 40%, by weight of the aqueous mixture;

(C) feeding the mixture of step (B) into a spray dryer and spray drying the mixture under conditions such that spheroidal microcapsules under about 150 microns in diameter are formed;

(D) providing the following ingredients of the coating layer, in percentages by weight of the coating layer;

(a) an edible material having a melting point of from about 25°C to about 100°C and selected from (i) fatty acids having an iodine value of from about 1 to about 10, (ii) natural waxes, (iii) synthetic waxes, and (iv) mixtures thereof, present in an amount of from about 80% to about 99.5%; and

(b) a surface active agent present in an amount of from about 0.5% to about 20%;

(E) preparing a homogeneous mixture of the ingredients in step (D); and

(F) coating the spray dried microcapsules from step (C) with the coating layer mixture from step (E) in a fluid bed dryer by suspending the microcapsules in a stream of air passing through a zone of the coating layer mixture under conditions such that spheroidal spray coated microcapsules under about 850 microns in diameter are formed.

18. A method according to claim 17, wherein the conditions in the coating step of step (F) include a coating spray rate of from about 2 ml/min. to about 10 ml/min.

19. A method according to claim 17 or 18, wherein the conditions in the ccating step of step (F) include a fluidizing air pressure of from about 1 atmosphere to about 5 atmospheres.

20. A method according to any of claims 17 to 19, wherein the conditions in the coating step of step (F) include a nozzle setting of from about 0.8 mm to about 2 mm.

21. A method according to any of claims 17 to 20, wherein the conditions in the coating step of step (F) include an outlet temperature of from about 20°C to about 50°C.

22. A method for preparing spheroidal coated capsules under 850 microns in diameter which comprise at least one spray dried spheroidal microcapsule core under about 150 microns in diameter and a coating layer over the core, which method comprises the step of:

(A) providing the following ingredients of the microcapsule core:

(a) a medicament present in an amount of from about 1% to about 90%, by weight of the core;

(b) a film forming polymer present in an amount of from about 8% to about 90%, by weight of the core; and

(c) a plasticizing agent in an amount of from about 5% to about 30%, by weight of the film forming

24

polymer;

(B) preparing an aqueous homogeneous mixture of the ingredients in step (A), wherein the medicament is present in a concentration of from about 0.25% to about 50%, and the film forming polymer is present in a concentration of from about 5% to about 40%, by weight of the aqueous mixture;

(C) feeding the mixture of step (B) into a spray dryer and spray drying the mixture under conditions such that spheroidal microcapsules under about 150 microns in diameter are formed;

(D) providing the following ingredients of the coating layer, in percentages by weight of the coating layer:

(a) an edible material having a melting point of from about 25°C to about 100°C and selected from (i) fatty acids having an iodine value of from about 1 to about 10, (ii) natural waxes, (iii) synthetic waxes, and (iv) mixtures thereof, present in an amount of from about 80% to about 99.5%; and

(b) a surface active agent present in an amount of from about 0.5% to about 20%;

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, feeding the dispersion into a heat controlled pressure spray nozzle, atomizing the dispersion under controlled conditions such that spheroidal spray congealed coated microcapsules under about 850 microns are formed.

23. A method for preparing spheroidal coated microcapsules under about 850 microns in diameter which comprise at least one spray dried spheroidal microcapsule core under about 150 microns in diameter and a coating layer over the core, which method comprises the steps of:

(A) providing the following ingredients of the microcapsule core:

(a) a medicament present in an amount of from about 1% to about 90%, by weight of the core;

(b) a film forming polymer present in an amount of from about 8% to about 90%, by weight of the core; and

(c) a plasticizing agent in an amount of from about 5% to about 30%, by weight of the film forming polymer; and

(B) preparing an aqueous homogeneous mixture of the ingredients in step (A), wherein the medicament is present in a concentration of from about 0.25% to about 50%, and the film forming polymer is present in a concentration of from about 5% to about 40%, by weight of the aqueous mixture;

(C) feeding the mixture of step (B) into a spray dryer and spray drying the mixture under conditions such that spheroidal microcapsules under about 150 microns in diameter are formed;

(D) providing the following ingredients of the coating layer, in percentages by weight of the coating layer:

(a) an edible material having a melting point of from about 25°C to about 100°C and selected from (i) fatty acids having an iodine value of from about 1 to about 10, (ii) natural waxes, (iii) synthetic waxes, and (iv) mixtures thereof, present in an amount of from about 80% to about 99.5%; and

(b) a surface active agent present in an amount of from about 0.5% to about 20%;

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, allowing the dispersion to cool, and pulverizing the cooled dispersion under conditions such that spheroidal coated microcapsules under about 850 microns in diameter are formed.

24. A method according to any of claims 17 to 23, wherein the medicament in the microcapsule core in step (A) is present in an amount of from about 1% to about 70%, by weight of the core.

25. A method according to any of claims 17 to 24, wherein the medicament in the microcapsule core in step (A) is a water-soluble medicament selected from dextromethorphan, dextromethorphan hydrobromide, pseudoephedrine, pseudoephedrine sulfate, pseudoephedrine hydrochloride, pseudoephedrine hydrobromide, chlorpheniramine maleate, guaidfenesin, diphenhydramine hydrochloride, and mixtures thereof.

26. A method according to any of claims 17 to 25, wherein the film forming polymer in the microcapsule core in step (A) is present in an amount of from about 20% to about 90%, by weight of the core.

27. A method according to any of claims 17 to 26, wherein the film forming polymer in step (A) is a cellulose derivative selected from cellulose acetate, cellulose acetate butyrate, cellulose acetate phthalate, ethylcellulose, hydroxypropylcellulose, hydroxypropylmethylcellulose, hydroxypropylmethylcellulose phthalate, methylcellulose, sodium carboxymethylcellulose, and mixtures thereof.

28. A method according to any of claims 17 to 27, wherein the edible material in the coating layer in step (D) is present in an amount of from about 85% to about 99.5%, by weight of the coating layer.

29. A method according to any of claims 17 to 28, wherein the edible material in the coating layer in step (D) is selected from hydrogenated palm oil, hydrogenated cottonseed oil, stearic acid, palmitic acid, and mixtures thereof.

30. A method according to any of claims 17 to 29, wherein the spheroidal coated microcapsules are under about 750 microns in diameter.

31. A method according to any of claims 17 to 30, wherein the weight ratio of microcapsule core to coating layer is from about 1:9 to about 9:1.

32. A chewable sustained release medicated composition, which comprises a pharmaceutically acceptable carrier and a therapeutically effective amount of spheroidal coated microcapsules according to any of claims 1 to 16.

33. A composition according to claim 32, wherein the coated microcapsules are present in the composition in an amount of from about 5% to about 50%, by weight of the composition.

34. A composition according to claim 32 or 33, wherein the pharmaceutically acceptable carrier is selected from a lozenge, a tablet, a toffee, a nougat, a suspension, and a chewy candy.

Claims for the following Contracting States: GR,ES

1. A method for preparing spheroidal coated microcapsules under about 850 microns in diameter which comprises at least one spray dried spheroidal microcapsule core under about 150 microns in diameter and a coating layer over the core, which method comprises the steps of:

(A) providing the following ingredients of the microcapsule core:

(a) a medicament present in an amount of from about 1% to about 90%, by weight of the core;

(b) a film forming polymer present in an amount of from about 8% to about 90%, by weight of the core; and

(c) a plasticizing agent in an amount of from about 5% to about 30%, by weight of the film forming polymer;

(B) preparing an aqueous homogeneous mixture of the ingredients in step (A), wherein the medicament is present in a concentration of from about 0.25% to about 50%, and the film forming polymer is present in a concentration of from about 5% to about 40%, by weight of the aqueous mixture;

(C) feeding the mixture of step (B) into a spray dryer and spray drying the mixture under conditions such that spheroidal microcapsules under about 150 microns in diameter are formed;

(D) providing the following ingredients of the coating layer, in percentages by weight of the coating layer:

(a) an edible material having a melting point of from about 25°C to about 100°C and selected from (i) fatty acids having an iodine value of from about 1 to about 10, (ii) natural waxes, (iii) synthetic waxes, and (iv) mixtures thereof, present in an amount of from about 80% to about 99.5%; and

(b) a surface active agent present in an amount of from about 0.5% to about 20%;

(E) preparing a homogeneous mixture of the ingredients in step (D); and

(F) coating the spray dried microcapsules from step (C) with the coating layer mixture from step (E) in a fluid bed dryer by suspending the microcapsules in a stream of air passing through a zone of the coating layer mixture under conditions such that spheroidal spray coated microcapsules under about 850 microns in diameter are formed.

2. A method according to claim 1, wherein the conditions in the coating step of step (F) include a coating spray rate of from about 2 ml/min. to about 10 ml/min.

3. A method according to claim 1 or 2, wherein the conditions in the coating step of step (F) include a fluidizing air pressure of from about 1 atmosphere to about 5 atmospheres.

4. A method according to any of claims 1 to 3, wherein the conditions in the coating step of step (F) include a nozzle setting of from about 0.8 mm to about 2 mm.

5. A method according to any of claims 1 to 4, wherein the conditions in the coating step of step (F) include an outlet temperature of from about 20°C to about 50°C.

6. A method for preparing spheroidal coated capsules under 850 microns in diameter which comprise at least one spray dried spheroidal microcapsule core under about 150 microns in diameter and a coating layer over the core, which method comprises the step of:

(A) providing the following ingredients of the microcapsule core:

(a) a medicament present in an amount of from about 1% to about 90%, by weight of the core;

(b) a film forming polymer present in an amount of from about 8% to about 90%, by weight of the core; and

(c) a plasticizing agent in an amount of from about 5% to about 30%, by weight of the film forming polymer;

(B) preparing an aqueous homogeneous mixture of the ingredients in step (A), wherein the medicament is present in a concentration of from about 0.25% to about 50%, and the film forming polymer is present

26

in a concentration of from about 5% to about 40%, by weight of the aqueous mixture;

(C) feeding the mixture of step (B) into a spray dryer and spray drying the mixture under conditions such that spheroidal microcapsules under about 150 microns in diameter are formed;

(D) providing the following ingredients of the coating layer, in percentages by weight of the coating layer:

(a) an edible material having a melting point of from about 25°C to about 100°C and selected from (i) fatty acids having an iodine value of from about 1 to about 10, (ii) natural waxes, (iii) synthetic waxes, and (iv) mixtures thereof, present in an amount of from about 80% to about 99.5%; and

(b) a surface active agent present in an amount of from about 0.5% to about 20%;

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, feeding the dispersion into a heat controlled pressure spray nozzle, atomizing the dispersion under controlled conditions such that spheroidal spray congealed coated microcapsules under about 850 microns are formed.

7. A method for preparing spheroidal coated microcapsules under about 850 microns in diameter which comprise at least one spray dried spheroidal microcapsule core under about 150 microns in diameter and a coating layer over the core, which method comprises the steps of:

(A) providing the following ingredients of the microcapsule core:

(a) a medicament present in an amount of from about 1% to about 90%, by weight of the core;

(b) a film forming polymer present in an amount of from about 8% to about 90%, by weight of the core; and

(c) a plasticizing agent in an amount of from about 5% to about 30%, by weight of the film forming polymer; and

(B) preparing an aqueous homogeneous mixture of the ingredients in step (A), wherein the medicament is present in a concentration of from about 0.25% to about 50%, and the film forming polymer is present in a concentration of from about 5% to about 40%, by weight of the aqueous mixture;

(C) feeding the mixture of step (B) into a spray dryer and spray drying the mixture under conditions such that spheroidal microcapsules under about 150 microns in diameter are formed;

(D) providing the following ingredients of the coating layer, in percentages by weight of the coating layer:

(a) an edible material having a melting point of from about 25°C to about 100°C and selected from (i) fatty acids having an iodine value of from about 1 to about 10, (ii) natural waxes, (iii) synthetic waxes, and (iv) mixtures thereof, present in an amount of from about 80% to about 99.5%; and

(b) a surface active agent present in an amount of from about 0.5% to about 20%;

(E) preparing a homogeneous mixture of the ingredients in step (D); and

(F) coating the spray dried microcapsules from step (C) with the coating layer mixture from step (E) by melting the coating layer mixture and dispersing the microcapsules uniformly therein, allowing the dispersion to cool, and pulverizing the cooled dispersion under conditions such that spheroidal coated microcapsules under about 850 microns in diameter are formed.

8. A method according to any of claims 1 to 7, wherein the medicament in the microcapsule core in step (A) is present in an amount of from about 1% to about 70%, by weight of the core.

9. A method according to any of claims 1 to 8, wherein the medicament in the microcapsule core in step (A) is a water-soluble medicament selected from dextromethorphan, dextromethorphan hydrobromide, pseudoephedrine, pseudoephedrine sulfate, pseudoephedrine hydrochloride, pseudoephedrine hydrobromide, chlorpheniramine maleate, guaidfenesin, diphenhydramine hydrochloride, and mixtures thereof.

10. A method according to claim 9, wherein the medicament is selected from pseudoephedrine sulfate, diphenhydramine hydrochloride, chlorpheniramine maleate, and mixtures thereof.

11. A method according to any of claims 1 to 10, wherein the film forming polymer in the microcapsule core in step (A) is present in an amount of from about 20% to about 90%, by weight of the core.

12. A method according to any of claims 1 to 11, wherein the film forming polymer in step (A) is a cellulose derivative selected from cellulose acetate, cellulose acetate butyrate, cellulose acetate phthalate, ethylcellulose, hydroxypropylcellulose, hydroxypropylmethylcellulose, hydroxypropylmethylcellulose phthalate, methylcellulose, sodium carboxymethylcellulose, and mixtures thereof.

13. A method according to claim 12, wherein the film forming polymer is ethyl cellulose.

14. A method according to any of claims 1 to 13, wherein the ingredients of the microcapsule core further comprises a dispersing agent present in an amount of up to about 10%, by weight of the core.

15. A method according to claim 14, wherein the dispersing agent is selected from aluminum stearate, kaolin, fumed silica, and mixtures thereof.

16. A method according to any of claims 1 to 15, wherein the edible material in the coating layer in step (D) is present in an amount of from about 85% to about 99.5%, by weight of the coating layer.

17. A method according to any of claims 1 to 16, wherein the edible material in the coating layer in step (D) is selected from hydrogenated palm oil, hydrogenated cottonseed oil, stearic acid, palmitic acid, and mixtures thereof.

18. A method according to claim 17, wherein the edible material is stearic acid.

19. A method according to any of claims 1 to 18, wherein the surface active agent in the coating layer is present in an amount of from about 0.5% to about 15%, by weight of the coating layer.

20. A method according to any of claims 1 to 19, wherein the surface active agent in the coating layer is selected from glyceryl monostearate, acetylated monoglycerides, stearic acid, and mixtures thereof.

21. A method according to any of claims 1 to 20, wherein the spheroidal coated microcapsules are under about 750 microns in diameter.

22. A method according to any of claims 1 to 21, wherein the weight ratio of microcapsule core to coating layer is from about 1:9 to about 9:1.

23. A process for preparing a chewable sustained release medicated composition, which comprises mixing a pharmaceutically acceptable carrier and a therapeutically effective amount of spheroidal coated microcapsules prepared by a method according to any of claims 1 to 22.

24. A process according to claim 23, wherein the coated microcapsules are present in the composition in an amount of from about 5% to about 50%, by weight of the composition.

25. A process according to claim 23 or 24, wherein the pharmaceutically acceptable carrier is selected from a lozenge, a tablet, a toffee, a nougat, a suspension, and a chewy candy.

# FIG. I

# FIG.2

# FIG.3

## DOCUMENTS CONSIDERED TO BE RELEVANT

EP 90308398.8

| Category | Citation of document with indication, where appropriate, of relevant passages | Relevant to claim | CLASSIFICATION OF THE APPLICATION (Int. Cl.5) |
|---|---|---|---|
| D,A | WO – A1 – 88/03 795 (A.M. MEHTA) * Claims; page 3, lines 14-27; page 9, line 34 – page 10, line 10; page 16, line 34 – page 19, line 1 * -- | 1,3,4, 6,7,9, 32-34 | A 61 K 9/50 A 61 K 9/52 |
| D,A | US – A – 4 764 380 (J. URQUHART et al.) * Totality * -- | 1,6,7, 11,14, 32,33 | |
| A | US – A – 4 016 254 (H. SEAGER) * Column 3, line 36 – column 12, line 27 * -- | 1-9, 17-21, 32-34 | |
| D,A | US – A – 4 749 575 (A. ROTMAN) * Column 3, line 41 – column 7, line 3 * ---- | 1, 32-34 | |
| | | | TECHNICAL FIELDS SEARCHED (Int. Cl.5)  A 61 K 9/00 |

The present search report has been drawn up for all claims

| Place of search | Date of completion of the search | Examiner |
|---|---|---|
| VIENNA | 18-12-1990 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)